# EUROPEAN PATENT SPECIFICATION

(11) **EP 1 247 524 B1**
(45) Date of publication and mention of the grant of the patent: **24.06.2015**
(21) Application number: 02252414.4
(22) Date of filing: 03.04.2002
(51) Int. Cl.: A61K 9/00, A61K 31/54, A61K 31/541

(54) **Composition comprising taurolidine and/or taurultam for the treatment of cancer**
Zubereitung enthaltend Taurolidin und/oder Taurultam zur Behandlung von Krebs
Composition comprenant taurolidine et/ou taurultam pour le traitement du cancer

(30) Priority: 03.04.2001 US 280748 P; 06.04.2001 US 281710 P; 06.04.2001 US 281711 P; 06.04.2001 US 281712 P; 06.04.2001 US 281713 P; 20.04.2001 US 284933 P; 20.04.2001 US 284934 P
(43) Date of publication of application: 09.10.2002
(60) Divisional application: 10009649.4
(73) Proprietor: ED. GEISTLICH SÖHNE AG FÜR CHEMISCHE INDUSTRIE, 6110 Wolhusen (CH)
(72) Inventor: Redmond, H. Paul, Wilton, Cork (IE); Stendel, Ruediger, 14163 Berlin (DE); Moehler, Hanns, 8706 Meilen (CH); Pfirrmann, Rolf W., 6006 Lucerne (CH)
(74) Representative: Matthews, Derek Peter

(56) References cited:
- EP-A- 1 066 830
- WO-A-01/39762
- WO-A-01/39763
- WO-A-91/13628
- WO-A-92/00743
- WO-A-98/52572
- WO-A-99/06114
- US-A- 5 593 665

## Description

The invention relates to the use of the methylol-containing compounds taurolidine and taurultam, for the manufacture of medicaments for the treatment of cancer.

Methylol transfer agents, such as the antibacterial and anti-toxin drug taurolidine and the related product taurultam, have been shown to exert a modifying effect on the toxicity of tumor necrosis factor (TNF) which is used, inter alia, in the treatment of tumors. Furthermore, the action of methylol transfer agents has been shown to be selective in that the growth of normal cell-lines was not significantly inhibited.

Taurolidine acts by transferring three methylol groups at the site of action, taurultam being an intermediate metabolite which itself transfers a single methylol group with liberation of the very well tolerated compound taurinamide. Thus, the two compounds act by essentially the same mechanism. It should be noted that methylol transfer is to be contrasted with methyl transfer which is characteristic of many highly toxic antitumor drugs. Taurolidine and taurultam have low toxicity and are not cytotoxic against normal cells.

Treatment of tumors with taurolidine and taurultam is disclosed in WO 91/13628, WO 92/00743, U.S. Patent No. 5 593 665, WO 98/52573, WO 99/06114, EP 1 066 830 A2, WO 01/39762 and WO 01/39763.

Programmed cell death is an evolutionary conserved biological principle in the regulation of cell numbers. Sensitive cells contain death receptors which are activated when the appropriate ligands are secreted from neighboring cells. A prominent system in programmed cell death is Fas-ligand mediated apoptosis. Fas, also known as CD 95/APO-1, is a cell surface receptor and a member of the tumor necrosis factor receptor superfamily which mediates apoptosis in sensitive cells upon oligomerization by the Fas-ligand (FasL).

In accordance with the present invention, a method of treating cancer is provided, whereby apoptotic death of a neoplastic cell is induced by contacting said cell with an apoptosis-inducing amount of a methylol-containing compound.

One embodiment comprises use of a methylol transfer agent in the manufacture of a medicament for use in inducing apoptotic death of a neoplastic cell in a mammal by contacting said cell with an apoptosis-inducing amount of said medicament, wherein said medicament is to be administered during at least two dosing cycles, each dosing cycle including (i) an administration phase of 1 to 8 days during which said medicament is administered each day at a total daily dosage of 2 to 60 g of said methylol transfer agent, and (ii) a non-administration phase of 1 to 14 days, during which no methylol transfer agent is administered, wherein the methylol transfer agent is taurolidine, taurultam or a mixture thereof.

In another embodiment, liver cancer is treated by intravenous infusion of solutions containing a methylol transfer agent, by direct administration through a catheter installed into a hepatic vessel, such as the hepatic artery, the portal vein, or the gastroduodenal artery.

In another embodiment, tumors of the central nervous system, such as glioma/glioblastoma, are treated.

The present invention relates to the ability of the methylol transfer agents, such as taurolidine, to induce cell toxicity, and to enhance Fas-ligand mediated apoptosis in combination therapy. Both taurolidine and its congener taurultam enhance the apoptotic effect of Fas-ligand in cancer cells at drug concentrations which per se show practically no effect on cell viability. In the human malignant glioma cell line LN-229 cell viability was reduced directly following incubation with taurolidine or taurultam alone. This effect enhanced the destruction of LN-229 cells by Fas-ligand. Thus, the use of methylol transfer agents to induce apoptotic cell death provides a means for treating cancer.

The two cell lines LN-18 and LN-229 represent validated model systems for apoptotic cell death with different sensitivities to Fas-ligand. These cell lines were therefore used to test the potential interaction of such compounds with the apoptotic pathway. The viability of the human malignant glioma cells LN-18 and LN-229 is differently affected by taurultam and taurolidine. The LN-18 cells, which are highly sensitive to Fas-ligand induced apoptosis, remained unaffected by taurultam at all concentrations tested (5, 20, 100 µg/ml) (Example 6). Taurolidine was able to only slightly reduce the viability of LN-18 cells at the highest concentration tested (100 µg/ml). Thus, the threshold for the destruction of LN-18 cells was reached at 0.01% of taurolidine. In contrast, LN-229 cells showed a much higher sensitivity to these drugs. In contrast to LN-18 cells, both taurultam and taurolidine by themselves (100 µg/ml) strongly decreased the viability of LN-229 cells.

Taurolidine (100 µg/ml) caused a dramatic death of LN-229 cells (70%) and taurultam (100 µg/ml) was able to reduce the viability of LN-229 cells by 30%. At the highest concentration tested (100 µg/ml), taurolidine alone was about as effective as the Fas-ligand in inducing cell death. Thus, taurolidine and taurultam have the ability to destroy human malignant cells.

The method is carried out by administering to a mammal suffering from cancer, compositions containing an active methylol-containing compound, at a dose sufficient to induce death of neoplastic cells by apoptosis. By "methylol-containing compound," or "methylol transfer agent," is meant a compound which contains or is capable of producing a methylol molecule under physiological conditions, specifically taurolidine and taurultam as disclosed in U.S. Patent No. 5 210 083 and mixtures thereof.

Treatment of an autologous tumor, e.g. a tumor of the central nervous system (CNS), is carried out by administering to a mammal, e.g. a human patient, a methylol-containing compound. The compound is administered systemically, e.g. orally or intravenously, or infused directly to the site of the tumor, e.g. to the brain or cerebrospinal fluid. An erodible or resorbable solid matrix such as a wafer or sponge can be implanted directly into brain tissue.

Cancers to which the present invention may be applicable include glioma, neuroblastoma, astrocytoma, carcinomatous meningitis, ovarian cancer, prostate cancer, central nervous system (CNS) cancer, lung cancer, gastric cancer, esophageal cancer, urinary bladder cancer, leukemia, lymphoma, melanoma, renal cell cancer and metastases thereof. Other cancers against which the method of the present invention is effective include other carcinomas, sarcomas or lymphomas, cancers of the head and neck, liver cancer, breast cancer and pancreatic cancer.

Particularly preferred embodiments involve treatment of cancers selected from the group consisting of glioma, neuroblastoma, astrocytoma, central nervous system (CNS) cancer, and liver cancer, as well as inhibition of tumor metastases thereof.

It is particularly beneficial to use taurolidine and/or taurultam, at concentrations sufficient to induce apoptosis in cancer cells, to prevent the spread of metastases, especially following surgical removal of tumors. The mammalian subjects are typically humans.

The invention also provides taurolidine, taurultam or a mixture thereof for use in inducing apoptotic death of a neoplastic cell in a mammal by contacting said cell with an apoptosis-inducing amount of said taurolidine compound, taurultam compound or mixture thereof, wherein said compound is to be administered during at least two dosing cycles, each dosing cycle including (i) an administration phase of 1 to 8 days during which said compound is administered each day at a total daily dosage of 2 to 60 g, and (ii) a non-administration phase of 1 to 14 days, during which no taurolidine compound, taurultam compound or mixture thereof is administered.

Effective dosage amounts of a methylol transfer agent in accordance with the present invention may comprise pharmaceutical dosage units within the range of 0.1-1,000 mg/kg, preferably 150-450 mg/kg per day, and most preferably 300-450 mg/kg per day. Alternatively, the dosages can be administered on a grams/day basis, from 2-60 g/day. Preferred doses may be in the range of 2-30 or 2.5-30 g/day taurolidine, 4-60 g/day taurultam, or a mixture thereof. Most preferred doses are in the range of 10-20 g/day taurolidine, 20-40 g/day taurultam, or a mixture thereof.

Suitable formulations for injection or infusion may comprise an isotonic solution containing one or more solubilizing agents, e.g. polyols such as glucose, in order to provide solutions of increased taurolidine or taurultam concentration. Such solutions are described in EP 253 662 B1. The concentration of taurolidine or taurultam in such solutions may be in the range 1-60 g/liter. Methylol transfer agents are generally poorly soluble in water. Thus, it is often required to administer relatively large volumes of aqueous solutions containing taurolidine or taurultam, for example 10g to 30g of taurolidine and/or taurultam. Preferred solutions for administration in accordance with the present invention contain from about 0.5-2% taurolidine and/or taurultam. It may be convenient to administer these compounds by infusion in view of the relatively large volumes concerned, conveniently at intervals throughout the day.

Administration, preferably by infusion, of the total daily dose can be carried out at a consistent rate over 24 hours, or according to a more rapid infusion schedule of the dose in portions, with breaks between each portion of the dose, e.g. infusion of 250 ml of a 2% taurolidine solution (5 g dose) over 2 hours, followed by a brief break of 4 hours, repeated over the course of a 24 hour infusion period to achieve a total daily dose of 20 g. Alternatively, 250 ml of a 2% taurolidine solution may be infused over one hour, with a one hour break between dose portions, and repeated until the daily dose is achieved, such that the total daily dose is provided over the course of less than 24 hours (i.e., approximately half the day), with no infusion occurring during the remainder of the day.

In accordance with one embodiment, four bottles (250 ml each) of 2% taurolidine solution are administered intravenously to patients with cancer, at a rate of 40 drops per minute, one bottle every six hours. The therapy cycle consists of an administration phase of daily infusions for one week, followed by a rest phase of two weeks. Total treatment consists of at least two such cycles. Efficacy of taurolidine 2% solution administered intravenously has been found to be particularly good with 25-28 bottles of 250 ml taurolidine 2% solution being instilled per cycle.

In accordance with a further embodiment of the invention, the administration phase comprises a daily regimen whereby 250 ml of taurolidine 2% solution is administered over the course of 2 hours, followed by a four hour break, repeated over 24 hours to achieve the total daily dose.

In accordance with a further embodiment of the invention, the administration phase comprises a daily regimen whereby 250 ml of 2% taurolidine solution is infused over one hour, followed by a one-hour break, and repeated until the daily dose is achieved. If the total dose is 20 g (for example), this regimen would provide the daily dose with four 250 ml infusions of 2% taurolidine over a 7 hour time span. No infusion occurs for the remainder of the day. Infusion rates can be lengthened (e.g., to 250 ml over 90 or 120 minutes) if the patient shows an elevated liver count.

In a further embodiment, concomitant administration of anti-convulsants and/or anti-oedema therapy and/or antibiotics and/or fluid and electrolyte replacement is carried out.

### 1. Anti-Convulsants

Preferably, the patient should be stabilized on anti-convulsive medications prior to treatment, to avoid complications during the treatment. This can conveniently be administered in part on an out-patient basis, as well as to prevent any emergency stabilization on an undesired medication. Valproinic acid is the agent of first choice; the dose should be determined in accordance with blood level checks and administered in 2 single doses. Normally, a dose of 1200 mg to 1500 mg is required. If a treatment with valproinic acid is not sufficient, a combination treatment with lamotrigin is possible. In case of allergies or if valproinic acid is not tolerated, the primary stabilization is to be done with lamotrigin. Phenytoin and carbamazepin are contraindicated.

### 2. Anti-Oedema Therapy

An anti-oedema therapy may also be administered, but only if absolutely necessary, because otherwise focal neurological symptoms may occur or become intensified, or intracerebral pressure may symptoms develop. Dexamethason should be given before or after the taurolidine was administered. The anti-oedema therapy should be administered with dexamethason, using the lowest possible dose. To protect the stomach a concomitant therapy with ranitidine 1 x 150 mg/day may be given. If stomach problems are observed with this therapy, an alternative treatment with antra 1-2 x 20 mg/day should be administered.

In cases of massively elevated intracerebral pressure and insufficient effectiveness of dexamethason, a therapy with mannitol, in particular at a dosage of up to 4 x 250 ml/day, is possible.

### 3. Antibiotic Therapy

A calculated antibiotic treatment with one of the subsequently listed antibiotics may be given, until the arrival of the sensitivity test.
* Urinary tract infection:
   primary: Cotrimoxazol
   alternative: Doxycyclin
* Pneumonia:
   primary: Erythromycin
   alternative: Doxycyclin
The following antibiotics should only be used if absolutely necessary (in the most severe, life-threatening infections) and if the sensitivity situation warrants it: Chino lone, penicillin, cephalosporin

### 4. Fluid and Electrolyte Replacement in Connection with Intravenous Taurolidine 2% Therapy

An amount of 250 ml of full electrolyte solution is preferably be given at the same time and with the same infusion speed parallel to the infusion with 250 ml taurolidine 2%. Electrolytes and blood count should be monitored twice per day, and the central vein pressure should be checked once daily.

If a hypernatraemia is observed, first, it should be determined whether dehydration is the cause. Diuretic agents should only be used if fluid is replaced at the same time and after dehydration was ruled out as the reason.

The methylol-containing compound is administered alone or in combination with one or more additional antineoplastic agents. In one preferred embodiment, the supplemental agent kills tumors cells by a mechanism other than apoptosis. For example, an antimetabolite, a purine or pyrimidine analogue, an alkylating agent, crosslinking agent (e.g., a platinum compound), and intercalating agent, and/or an antibiotic is administered in a combination therapy regimen. The supplemental drug is given before, after, or simultaneously with the methylol-containing agent. For example, the methylol transfer agent can be co-administered with a fluoro-pyrimidine, such as 5-fluoro-uracil (5-FU). Effective daily dosage amounts of a fluoro-pyrimidine may be in the range of about 0.1-1,000 mg per pharmaceutical dosage unit. Effective dosage amounts of 5-FU also may be in the range of about 100-5,000 mg/m² body surface area, preferably about 200-1,000 mg/m² body surface area, more preferably about 500-600 mg/m² body surface area. 5-FU typically is provided in 250 mg or 500 mg ampules for injection, or 250 mg capsules for oral administration.

In another embodiment, the apoptotic effect of methylol transfer agents can be enhanced by co-administration with a Fas-ligand. A Fas-ligand polypeptide is disclosed in U.S. Patent No. 5,858,990. Therapeutically effective amounts of Fas-ligand generally will be within a range of about 0.01-1,000 mg/kg patient body weight, preferably about 0.1-200 mg 1 kg patient body weight, most preferable about 0.2-20 mg/kg patient body weight. The therapeutically effective amounts can be administered as dosages once per day, or multiple times per day such as two, three, four or more times per day.

In LN-18 cells taurultam (100 µg/ml) clearly enhanced apoptosis induced by 0.4 or 2.0 vol.% Fas-ligand. Example 1. This is the more striking as taurultam by itself did not impair the cell viability at this concentration. Thus, taurultam is able to enhance the effectiveness of the Fas-ligand induced apoptotic pathway. The same holds for taurolidine (100 µg/ml), although taurolidine alone did reduce cell viability at this concentration. Example 1. These results support the view that the apoptotic affect of taurultam and taurolidine is enhanced by Fas-ligand. When taurultam or taurolidine at a concentration of 100 µg/ml are combined with Fas-ligand, the total cell loss represents itself as the sum of that of Fas-ligand and of taurolidine or taurultam alone. Thus, the cytotoxicity of taurultam and taurolidine at this concentration appears to be additive to the Fas-mediated apoptosis. At lower concentrations, the apoptopic effect of taurolidine and taurultam are greatly enhanced, beyond an additive effect, by co-administration with the Fas-ligand.

The invention also includes treating a drug resistant tumor, e.g., a multiple drug resistant (MDR) tumor, in a mammal by administering to the mammal a methylol-containing compound. The tumor to be treated is a carcinoma or sarcoma. The drug resistant tumor is selected from the group consisting of a solid tumor, a non-solid tumor, and a lymphoma. For example, the drug resistant tumor is a breast cancer, ovarian cancer, colon cancer, prostate cancer, pancreatic cancer, CNS cancer, liver cancer, lung cancer, urinary bladder cancer, lymphoma, leukemia, or sarcoma.

According to another embodiment, a solution containing taurolidine and/or taurultam further contains taurin, in an amount within a range of about 1-20 g/l, preferably about 5 g/l.

A further embodiment provides methods for treating both primary liver tumors and metastases thereof, by direct administration of a solution containing a methylol transfer agent to the liver through a catheter installed in a hepatic vessel. By administering the methylol transfer agent in a solution that assists in maintaining liver function and non-ischemic conditions, therapy is directed to the affected organ, without unduly subjecting the organ to undue stress.

For treatment of primary liver tumors, the solution of methylol transfer agent may be administered through the hepatic artery, such that the therapeutic agent is carried into the organ for maximum effect. Alternatively, the solution can be supplied via the gastroduodenal artery, for delivery to the liver through the hepatic artery. The preferred solution for use in this embodiment is one that assists in maintaining liver function and minimizing stress to the organ associated with infusion of large volumes of methylol transfer agent solution. Solutions which may be used in the present invention are set forth in the Examples.

### Example 1: Isotonic Solution 2% Taurolidine

One suitable composition for intravenous drop infusion is shown below.
Isotonic sterile solution, 100 ml:

| | |
|---|---|
| 2.0 g | Taurolidine |
| 5.0 g | PVP 16 PF UP aqua dest. ad solut. 100 ml. PH 7.2 - 7.3 |

Sterile-filtered and steam sterilization.

### Example 2: Isotonic Taurolin® Solution 2% Taurolidine with Taurin and electrolytes

Another suitable composition for intravenous drop infusion is shown below.
Isotonic sterile solution, 100 ml:
   2.0 g Taurolidine
   5.0 g PVP 17 PF UP
   0.5 g Taurin
   0.3 g Sodium chloride
Sterile-filtered and steam sterilization

### Example 3: Isotonic Taurolin® Ringer Solution 2% Taurolidine with Taurin and electrolytes

Another suitable composition for intravenous drop infusion is shown below.
Isotonic sterile solution, 100 ml:
   2.0 g Taurolidine
   5.0 g PVP 17 PF UP
   0.5 g Taurin
   0.26 g Sodium chloride
   0.0033 g Potassium chloride
   0.004 g Calcium chloride 2H₂O
   0.003 g Sodium hydrogen carbonate
Sterile-filtered and steam sterilization

### Example 4: Taurolin® Ringer-Lactate 2% Taurolidine with Taurin and electrolytes

Another suitable composition for intravenous drop infusion is shown below.
Isotonic sterile solution, 100 ml:
   2.0 g Taurolidine
   5.0 g PVP 17 PF UP
   0.5 g Taurin
   0.20 g Sodium chloride
   0.013 g Potassium chloride
   0.009 g Calcium chloride 2H₂O
   0.0033 g Sodium lactate 50% solution (Pharmacopeia Europea)
Sterile-filtered and steam sterilization

### Example 5: Taurultam Solution

One preferred solution comprises:

| | |
|---|---|
| Lactobionic acid | 35.830 g |
| Adenosine | 1.340 g |
| Raffinose Pentahydrate | 17.830 g |
| Hydroxyethyl starch (HES) PL 40/0.5 | 50.000 g |
| Glutathione | 0.929 g |
| Allopurinol | 0.136 g |
| Taurultam | 10.000 g |
| Kcl | 5.200 g |
| MgSO₄ 7H₂O | 1.230 g |
| NaOH 25% GV to pH 7.8 NaOH pellets Merck 6482 | |
| Distilled water | 900 ml |

The solution was sterilized from 16 minutes at 121° C. The pH after sterilization was 7.2, and pH of ready to use solution was 7.47.

### Example 6: Inducement of apoptosis

Taurolidine and taurultam were tested for their ability to enhance apoptosis or induce cell death, alone and in combination with the Fas-ligand, in human malignant glioma cell lines. The two cell lines LN-18 and LN-229 represent validated model systems for apoptotic cell death with different sensitivities to Fas-ligand (Schlappbach and Fontana, 1997). These cell lines were therefore used to test the potential interaction of taurultam or taurolidine with the apoptotic pathway.

### 1) Reagents

Taurolidine (Batch Nr. 41692/7) and taurultam (Batch E/39024/4) were provided by Geistlich Pharma AG, Wolhusen, Switzerland. DME-Culture Medium and fetal bovine serum (FBS) were purchased from Gibco BRL, Basel, Switzerland. The cell proliferation assay WST-1 was purchased from Roche Diagnostics, Rotkreuz, Switzerland. Fas-ligand (supernatant from an overexpression system) and the human glioma cell lines LN-18 and LN-229 were kindly provided by Prof. A. Fontana, Institute of Clinical Immunology, University Hospital, Zurich, Switzerland

### 2) Cell lines

The cell lines LN-18 and LN-229 were cultured at 37°C and 5% CO₂ in DMEM containing 5% FBS and 2 mM glutamin (10 cm plates NUNCLON 15035). In the experiments in which Fas-ligand was tested by itself , about 1x10⁴ cells were plated per well in 96-well plates (NUNCLON 167008) resulting in a confluency of about 60% on the following day (17h incubation). In all other experiments about 1.5x10⁴ cells were plated which resulted in a confluency of about 90% on the following day (17h incubation). Fas-ligand was added as supernatant indicated as % volume (vol%) of total culture volume.

### 3) Cell viability test

LN-18 and LN-229 cells were incubated in 50 µl medium in the absence or presence of either Fas-ligand, taurultam, taurolidine or respective combinations thereof. After a 17h incubation the cell viability was determined by adding 50 µl medium containing a double concentrated WST-1 reagent. The coloration resulting from the activity of the mitochondrial succinate reductase, was measured in an ELISA reader at 450 nm using a reference wavelength of 690 nm.

The human malignant glioma cell lines LN-18 and LN-229 were used to test the ability of taurolidine and taurultam to affect cell viability and/or to enhance Fas-ligand induced apoptosis. The two human malignant glioma cell lines, LN-18 and LN-229 had previously been reported to display different sensitivity to the apoptotic effect of Fas-ligand (Schlappbach and Fontana, 1997).

### 1) Sensitivity of LN-18 and LN-229 to Fas-ligand

In a first set of experiments it was investigated whether the different sensitivity of LN-18 and LN-229 to Fas-ligand was reproduced under our experimental conditions. The two cell lines were incubated over night (17h) in 96 well plates containing 1x10⁴ cells per well with increasing concentrations of Fas-ligand (3.1, 6.25, 12.5, 25.0 and 50 vol.%). In the absence of Fas-ligand the cells reached about 60 % confluency after overnight incubation. In the presence of Fas-ligand LN-18 was extremely sensitive, displaying more than 90% loss of cell viability in the presence of only 6.25 vol. % Fas-ligand. Even at 3.1%, an approximately 85% reduction in cell viability was observed. In contrast, the viability of LN-229 cells was not greatly affected by 6.25 vol. % Fas-ligand (approximately 10% reduction) and was reduced only at higher concentrations with a maximum of 40% cell loss in the presence of the highest concentration of Fas-ligand tested (50 vol. %).

### 2) Influence of taurultam on Fas-ligand induced apoptosis in LN-18-cells

LN-18 cells were incubated for 17h with increasing concentrations of taurultam (5, 20, 100 µg/ml) in the absence and presence of two concentrations of Fas-ligand (0.4 vol. % and 2.0 vol. %). Taurultam by itself even at the highest concentration tested (100 µg/ml) did not affect the cell viability (an approximately 5% reduction was observed at 5 and 20 µg/ml, and viability actually appeared to increase at 100 µg/ml). In the presence of 0.4 vol.% Fas-ligand alone cell viability was reduced by only about 10%, an effect which remained unchanged in the presence of 5 or 20 µg/ml taurultam. However cell viability was strongly decreased when 0.4 vol.% Fas-ligand was coincubated with of 100 µg/ml taurultam. When the Fas-ligand was added at a higher concentration (2.0 vol. %) apoptosis was induced in 60% of the cells by Fas-ligand alone. This effect was also increased by taurultam at 100 µg/ml but not at 5 or 20 µg/ml. Thus, taurultam is able to enhance the apoptotic effect of Fas-ligand in LN-18 cells at a concentration (100 µg/ml) which by itself did not affect cell viability.

### 3) Influence of taurolidine on Fas-ligand induced apoptosis in LN-18 cells

LN-18 cells were incubated for 17h with either 0.4 or 2.0 vol.% Fas-ligand in the absence and presence of increasing concentrations of taurolidine (5, 20, 100 µg/ml). Taurolidine by itself did not appreciably affect cell viability yielding a reduction by only 10% at the highest concentration tested (100 µg/ml). In the presence of Fas-ligand alone (0.4% or 2.0%) the cell viability was affected in the same way as described above. The cell viability was further reduced by taurolidine but only at the highest concentration tested (100 µg/ml). Thus, taurolidine was able to enhance the effect of Fas-ligand on LN-18 cells at a concentration (100 µg/ml) which did not appreciably affect cell viability per se.

### 4) Influence of taurultam on Fas-ligand induced apoptosis in LN-229 cells

The incubation of LN-229 cells for 17h with taurultam alone had no effect at 5 and 20 µg/ml but reduced cell viability by 35% at 100 µg/ml. When the LN-229 cells were incubated with Fas-ligand alone (10% or 50%) the cell viability was reduced by only about 20% in the presence of a high concentration of Fas-ligand (50 vol. %). When taurultam was added at concentrations which were inactive per se (5 and 20 µg/ml) no change in the effectiveness of the Fas-ligand (10 or 50 vol.%) was observed. It was only at the highest concentration of taurultam (100 µg/ml) that Fas-ligand induced cell loss was further enhanced. Thus, the results with LN-229 demonstrate the ability of taurultam to enhance the destruction of cells in the presence of Fas-ligand.

### 5) Influence of taurolidine on Fas-ligand induced apoptosis in LN-229 cells

The exposure of LN-229 cells to taurolidine alone for 17h caused a strong loss of cell viability by about 70% at the highest concentration tested (100 µg/ml). Thus, LN-229 cells were more sensitive to taurolidine than LN-18 cells. When coincubated with Fas-ligand (10 vol.%) cell destruction was enhanced by taurolidine at 100 µg/ml. At 50 vol.% Fas-ligand the effect was more pronounced and apparent even for taurolidine 20 µg/ml.

### Example 7: Use and application of taurolidine and/or taurultam for the treatment and/or prophylaxis of tumors of the central nervous system

### 1. Tumor cells used for the experiments

For experiments, C6 glial tumor cells, HT22 neuronal tumor cells, U373 human glioma/glioblastoma tumor cells and cells derived from patients with glioblastoma were used.

### 2. Preparation of patient-derived tumor cells

Tumor cells derived from patients with glioblastoma were obtained intraoperatively. Tumor tissue was stored in RPMI 1640 medium without FCS. Tissue was then sub cultured in 15 ml Falcon flasks; adding 0.025% trypsin with PBS, followed by incubation at 37°C. After this, RPMI 1640 with FCS was added and centrifugation performed. The next step was incubation with DNAse, resuspension and dissociation, followed by washing step in medium to remove DNAse. Cells were then cultured in Falcon flasks.

### 3. Method of anti-neoplastic action of Taurolidine and/or Metabolites

Ultrastructurally, shrinkage of cytoplasm, condensation and marginalization of chromatin could be observed. These changes were already apparent at 30 minutes of incubation with 0.1 µg/ml taurin and increased strikingly over time and with concentration of taurolidine. Mitochondria were not affected ultrastructurally. Flow cytometry showed an initial increase in the GO/G1 peak and S-phase starting at 30 minutes. These initial changes were followed by a decrease in forward light and side scatter. In addition, concentration-dependent fragmentation of DNA started at 60 minutes. Following 24 hours, fragmentation of the DNA was nearly complete. At concentrations of 2.0 µg/ml taurolidine and more, the changes in cell size was only marginal.

The described results in combination with the results of special dying methods (Leucostat preparation) suggests an apoptotic mechanism of tumor cell death. Normal brain cells were not affected by incubation with taurolidine or taurultam in concentrations of up to 4 µg/ml for up to 5 days.

### Example 8: Two-cycle dosing Schedule for Treating Patients with Cancer Using Intervenous Taurolidine 2%

Four bottles (250 ml each) of 2% taurolidine solution are administered intravenously to patients with cancer, at a rate of 40 drops per minute, one bottle every six hours. The dosing cycle consists of an administration phase of daily infusions for one week, followed by a non-administration phase of two weeks, then followed by another administration phase of four bottles per day as previously indicated. Efficacy of taurolidine 2% solution administered intravenously has been found to be particularly good with 25-28 bottles of 250 ml taurolidine 2% solution being instilled per cycle.

### Example 9: Four-cycle Dosing Schedule for Treating Patients with Malignant Gliomas Using Intravenous Taurolidine 2%

The treatment comprises a minimum of 4 cycles. Each cycle is 7 days long, and is comprised as follows:
1. First Cycle
   a. Intravenous infusion of 250 ml taurolidine 2% and 250 ml full electrolyte solution via the central vein catheter with an infusion time of 60 minutes.
   b. If this therapy causes an elevated liver count, it is necessary to increase the infusion time to 90 or 120 minutes.
   c. 60-minute break
   d. Repeat the therapies under a or b and c for a total of 6 times per day.
   e. At an infusion time of 60 minutes the duration of the daily infusion program per 250 ml of taurolidine is 11 hours, at 90 minutes of infusion time 14 hours, and at 120 minutes of infusion time 17 hours. No drug is administered for the remainder of the time.
   f. rest phase
2. Subsequent Cycles
   a. Intravenous infusion of 250 ml taurolidine 2% and 250 ml full electrolyte solution via the central vein catheter with an infusion time of 60 minutes.
   b. If this therapy causes an elevated liver count, it is necessary to increase the infusion time to 90 or 120 minutes.
   c. 60 minute break
   d. Repeat the therapies under a or b and c for a total of 4 times per day.
   e. At an infusion time of 60 minutes the duration of the daily infusion program per 250 ml of taurolidine is 7 hours, at 90 minutes of infusion time 9 hours, and at 120 minutes of infusion time 11 hours. No drug is administered for the remainder of the time.

### Example 10: Therapy of Glioblastoma with Taurolidine (Single Case Observation)

The following is a case involving treatment of a single individual with a single treatment cycle.

Patient: "F.D.," male, 59 years

Diagnosis: large (8 x 8 x 8 cm) malignant glioma bifrontal with affection of the corpus callosum ("butterfly glioma").

Procedure prior to treatment with taurolidine: Patient was referred to Neurosurgical departments in Heidelberg and Wurzburg, operation was refused, radiation and chemotherapy were refused by the patient.

Prior treatment: oral corticosteroids.

Planned Treatment: Taurolidine intravenously

Chief complaints on admission: Diffuse headache, urinary incontinence, blurred vision, motor aphasia, gait disturbance, impaired memory.

Neurological examination on admission: Awake -somnolent, alert, impaired vision, nearly complete motor aphasia, apraxia, gait disturbance, urinary incontinence, severe mnesic and concentration deficits

Karnofsky index on admission: 20 -30

MRI at Day 1 of treatment (pre treatment): Bifrontal space occupying lesion (ca. 8 x 8 x 8 cm) with irregular shape and ring like contrast enhancement and destructive affection of the corpus callosum. The marked space occupying effect leads to disappearance of nearly all reserve spaces.

### Treatment

Day 1: Informed consent; Blood samples; MRI.
Day 2: Insertion of a central venous line; Chest X-ray.
Days 3-8: Intravenous administration of 4 x 250 ml of 2 % taurolidine/day within 2 hours, followed by an interval of 4 hours; Blood samples twice daily; Substitution of electrolytes.
Day 9: Intravenous administration of 1 x 250 ml of 2 % Taurolidine within 2 hours; Discharge.

### Treatment summary:

In total, 25 x 250 ml of 2 % taurolidine (125 g taurolidine) were administered without side effects. Electrolytes and fluid were substituted according to the results of the blood samples.

Chief complaints on discharge: Headache improved, no urinary incontinence, vision improved, gait disturbance improved, motor aphasia slightly improved, impaired memory.

Neurological examination on discharge: Awake, alert, vision improved, motor aphasia slightly improved, gait disturbance improved, apraxia slightly improved, no urinary incontinence, severe mnesic and concentration deficits

Karnofsky index on discharge: 40-50

In view of the dramatic improvement observed in the patient's condition after a single treatment cycle, it is expected that an infusion regime of at least two cycles will provide the desired therapeutic effect.

### Example 11: Treatment of Severe Glioblastoma Multiforme Grade IV

Prior to treatment the patient exhibited severe glioblastoma multiforme grade IV, left temporal lobe affected. The tumor was prominent in computer tomography pictures of the patient's cranium, prior to treatment. The patient's cranium was imaged in a T2-weighted picture sequence in axial, sagittal and coronary layer orientation as well as T1-weighted picture sequence in axial layer orientation natively and in axial, coronary and sagittal layer orientation after contrast medium application as well as MR spectroscopy.

The patient was treated with four treatment cycles each consisting of a seven-day infusion phase of a daily dose of 20 g taurolidine (4 x 250 ml 2% taurolidine solution) and a two-day rest phase. After the four cycles, the patient underwent an additional two-day infusion phase. Regular computer tomography images of the patient's cranium were taken during treatment.

By the end of the second treatment cycle (200 g taurolidine administered), brain edema was noticeably reduced. By the end of third treatment cycle (300g taurolidine administered), tumor growth had stopped. After the completion of the entire course of treatment (600g taurolidine administered), the tumor was shown by computer tomography to be almost completely disintegrated. Little or no necrosis was observed during the course of treatment, indicating that the tumor reduction was the result of apoptosis.

### Example 12: Treatment of brain tumors with direct application of taurolidine/taurultam

The methylol transfer agent is applied directly to the tumor cavity using taurolidine/taurultam containing tubes consisting of several segments with semipermeable membrane.

Following total or partial tumor removal, a special tube is implanted in the tumor cavity, so that the end of this tube lies subgaleal. The tube includes various segments of semipermeable material, which contains taurolidine/taurultam and can be refilled via a subgaleal port.

## Claims

1. Use of a methylol transfer agent in the manufacture of a medicament for use in inducing apoptotic death of a neoplastic cell in a mammal by contacting said cell with an apoptosis-inducing amount of said medicament, wherein said medicament is to be administered during at least two dosing cycles, each dosing cycle including (i) an administration phase of 1 to 8 days during which said medicament is administered each day at a total daily dosage of 2 to 60 g of said methylol transfer agent, and (ii) a non-administration phase of 1 to 14 days, during which no methylol transfer agent is administered, wherein the methylol transfer agent is taurolidine, taurultam or a mixture thereof.

2. Use as claimed in claim 1, wherein the methylol transfer agent is taurolidine.

3. Use as claimed in claim 2, wherein the medicament is to be administered during each administration phase at a total daily dosage of 2 to 30 g of taurolidine.

4. Use as claimed in claim 1, wherein the methylol transfer agent is taurultam.

5. Use as claimed in claim 4, wherein the medicament is to be administered during each administration phase at a total daily dosage of 4 to 60 g of taurultam.

6. Use as claimed in any preceding claim, wherein the medicament is to be administered during each administration phase in a dosage which provides 150 to 450 mg/kg per day of said methylol transfer agent.

7. Use as claimed in claim 6 wherein the medicament is to be administered during each administration phase in a dosage which provides 300 to 450 mg/kg per day of said methylol transfer agent.

8. Use as claimed in any preceding claim, wherein the medicament is to be coadministered with a second antineoplastic agent.

9. Use as claimed in claim 8, wherein the second antineoplastic agent is 5-fluorouracil.

10. Use as claimed in claim 9, wherein the 5-fluorouracil is to be administered in an amount per day of 100 to 5,000 mg/m²body surface area.

11. Use as claimed in claim 10, wherein the 5-fluorouracil is to be administered in an amount per day of 200 to 1,000 mg/m²body surface area.

12. Use as claimed in any one of claims 1 to 7, wherein the medicament is to be coadministered with Fas-ligand.

13. Use as claimed in claim 12, wherein the Fas-ligand is to be administered in an amount per day of 0.01 to 1,000 mg/kg body weight.

14. Use as claimed in any preceding claim, wherein the medicament comprises a solution of the methylol transfer agent, said solution further containing taurin.

15. Use as claimed in claim 14, wherein said taurin is present in said solution at a concentration in the range 1 to 10 g/l.

16. Use as claimed in any preceding claim, wherein the medicament is for use in the treatment or prophylaxis of liver cancer.

17. Use as claimed in claim 16, wherein the medicament is to be administered directly to the liver via a hepatic vessel.

18. Use as claimed in claim 16, wherein the medicament is to be administered via a hepatic artery or a gastroduodenal artery in the treatment of a primary liver tumour.

19. Use as claimed in claim 17, wherein the medicament is to be administered via a portal vein in the treatment of metastatic liver cancer.

20. Use as claimed in any preceding claim, wherein the medicament is to be administered during each administration phase by continuous infusion such that the daily dosage of the methylol transfer agent is infused over 24 hours.

21. Use as claimed in any one of claims 1 to 19, wherein the medicament is to be administered during each administration phase by infusion such that the daily dosage of the methylol transfer agent is infused as a series of partial doses, each partial dose infusion being followed by a break during which no infusion occurs.

22. Use as claimed in claim 21, wherein the partial doses are to be infused such that the total daily dosage of the methylol transfer agent will be administered over a course of 24 hours.

23. Use as claimed in claim 22, wherein each partial dose is to be infused over a course of two hours, followed by a break of four hours.

24. Use as claimed in claim 21, wherein the partial doses are to be infused such that the total daily dosage of the methylol transfer agent will be administered over a course of less than 24 hours.

25. Use as claimed in claim 24, wherein each partial dose is to be infused over a course of one hour, followed by a break of one hour.

26. Use as claimed in any preceding claim, wherein said medicament is to be coadministered with at least one supplemental agent selected from anti-convulsants, anti-oedema agents, antibacterial agents and electrolyte solutions.

27. Use as claimed in claim 26, wherein the supplemental agent is an electrolyte solution.

28. Use as claimed in any preceding claim, wherein the medicament is to be administered in two dosing cycles.

29. Use as claimed in claim 28, wherein each dosing cycle comprises (i) a seven day administration phase during which four 250 ml dosage portions of 2% taurolidine solution are to be administered as a continuous infusion over each of the seven days, and (ii) a seven day non-administration phase.

30. Use as claimed in any one of claims 1 to 27, wherein the medicament is to be administered in four dosing cycles.

31. Use as claimed in claim 30, wherein each dosing cycle includes a seven day administration phase, during each day of which four 250 ml dosage portions of 2% taurolidine solution are to be administered such that each dosage portion is to be infused over the course of one to two hours, followed by a non-administration break of one hour.

32. Taurolidine, taurultam or a mixture thereof for use in inducing apoptotic death of a neoplastic cell in a mammal by contacting said cell with an apoptosis-inducing amount of said taurolidine compound, taurultam compound or mixture thereof, wherein said compound is to be administered during at least two dosing cycles, each dosing cycle including (i) an administration phase of 1 to 8 days during which said compound is administered each day at a total daily dosage of 2 to 60 g, and (ii) a non-administration phase of 1 to 14 days, during which no taurolidine compound, taurultam compound or mixture thereof is administered.

33. Taurolidine, taurultam or a mixture thereof as claimed in claim 32, for use in inducing apoptotic death of a neoplastic cell in a mammal according to claim 32, wherein said contacting with said neoplastic cell is as defined in any one of claims 2 to 31.

## Patentansprüche

1. Gebrauch eines Methyloltransfermittels bei der Herstellung eines Medikaments zum Gebrauch beim Einleiten von apoptotischem Tod einer neoplastischen Zelle in einem Säuger durch Inberührungbringen der Zelle mit einer Apoptose einleitenden Menge des Medikaments, wobei das Medikament in wenigstens zwei Dosierzyklen zu verabreichen ist, wobei jeder Dosierzyklus (i) eine Verabreichungsphase von 1 bis 8 Tagen, in der das Medikament täglich mit einer Tagesgesamtdosis von 2 bis 60 g des Methyloltransfermittels verabreicht wird, und (ii) eine Nichtverabreichungsphase von 1 bis 14 Tagen, in denen kein Methyloltransfermittel verabreicht wird, umfasst, wobei das Methyloltransfermittel Taurolidin, Taurultam oder ein Gemisch aus diesen ist.

2. Gebrauch nach Anspruch 1, wobei das Methyloltransfermittel Taurolidin ist.

3. Gebrauch nach Anspruch 2, wobei das Medikament in jeder Verabreichungsphase mit einer Tagesgesamtdosis von 2 bis 30 g Taurolidin zu verabreichen ist.

4. Gebrauch nach Anspruch 1, wobei das Methyloltransfermittel Taurultam ist.

5. Gebrauch nach Anspruch 4, wobei das Medikament in jeder Verabreichungsphase mit einer Tagesgesamtdosis von 4 bis 60 g Taurultam zu verabreichen ist.

6. Gebrauch nach einem der vorhergehenden Ansprüche, wobei das Medikament in jeder Verabreichungsphase in einer Dosis zu verabreichen ist, die 150 bis 450 mg/kg des Methyloltransfermittels pro Tag bereitstellt.

7. Gebrauch nach Anspruch 6, wobei das Medikament in jeder Verabreichungsphase in einer Dosis zu verabreichen ist, die 300 bis 450 mg/kg des Methyloltransfermittels pro Tag bereitstellt.

8. Gebrauch nach einem der vorhergehenden Ansprüche, wobei das Medikament zusammen mit einem zweiten antineoplastischen Mittel zu verabreichen ist.

9. Gebrauch nach Anspruch 8, wobei das das zweite antineoplastische Mittel 5-Fluoruracil ist.

10. Gebrauch nach Anspruch 9, wobei das 5-Fluoruracil in einer täglichen Menge von 100 bis 5.000 mg/m² Körperoberfläche zu verabreichen ist.

11. Gebrauch nach Anspruch 10, wobei das 5-Fluoruracil in einer täglichen Menge von 200 bis 1.000 mg/m² Körperoberfläche zu verabreichen ist.

12. Gebrauch nach einem der Ansprüche 1-7, wobei das Medikament zusammen mit einem Fas-Liganden zu verabreichen ist.

13. Gebrauch nach Anspruch 12, wobei der Fas-Ligand in einer täglichen Menge von 0,01 bis 1.000 mg/kg Körpergewicht zu verabreichen ist.

14. Gebrauch nach einem der vorhergehenden Ansprüche, wobei das Medikament eine Lösung des Methyloltransfermittels enthält, wobei die Lösung ferner Taurin enthält.

15. Gebrauch nach Anspruch 14, wobei das Taurin in einer Konzentration im Bereich von 1 bis 10 g/l in der Lösung vorliegt.

16. Gebrauch nach einem der vorhergehenden Ansprüche, wobei das Medikament für den Gebrauch bei der Behandlung von oder der Prophylaxe gegen Leberkrebs eingesetzt wird.

17. Gebrauch nach Anspruch 16, wobei das Medikament über ein hepatisches Blutgefäß direkt in die Leber verabreicht wird.

18. Gebrauch nach Anspruch 16, wobei das Medikament bei der Behandlung eines Primärtumors der Leber über eine Leberarterie oder über eine Gastroduodenalarterie verabreicht wird.

19. Gebrauch nach Anspruch 17, wobei das Medikament bei der Behandlung eines metastatischen Leberkrebses über eine Pfortader verabreicht wird.

20. Gebrauch nach einem der vorhergehenden Ansprüche, wobei das Medikament in jeder Verabreichungsphase durch Dauerinfusion zu verabreichen ist, sodass die Tagesdosis des Methyloltransfermittels über 24 Stunden infundiert wird.

21. Gebrauch nach einem der Ansprüche 1 bis 19, wobei das Medikament in jeder Verabreichungsphase durch Infusion derart zu verabreichen ist, dass die Tagesdosis des Methyloltransfermittels als eine Reihe von Teildosen infundiert wird, wobei jede Teildosisinfusion von einer Pause gefolgt wird, in der keine Infusion stattfindet.

22. Gebrauch nach Anspruch 21, wobei die Teildosen derart zu infundieren sind, dass die Tagesgesamtdosis des Methyloltransfermittels über einen Zeitraum von 24 Stunden verabreicht wird.

23. Gebrauch nach Anspruch 22, wobei jede Teildosis über einen Zeitraum von zwei Stunden, gefolgt von einer Pause von vier Stunden zu infundieren ist.

24. Gebrauch nach Anspruch 21, wobei die Teildosen derart zu infundieren sind, dass die Tagesgesamtdosis des Methyloltransfermittels über einen Zeitraum von weniger als 24 Stunden verabreicht wird.

25. Gebrauch nach Anspruch 24, wobei jede Teildosis über einen Zeitraum von einer Stunde, gefolgt von einer Pause von einer Stunde zu infundieren ist.

26. Gebrauch nach einem der vorhergehenden Ansprüche, wobei das Medikament zusammen mit dem wenigstens einem Hilfsmittel zu verabreichen ist, welches ausgewählt ist aus krampflösenden Mitteln, Anti-Ödem-Mitteln, antibakteriellen Mitteln und Elektrolytlösungen.

27. Gebrauch nach Anspruch 26, wobei das Hilfsmittel eine Elektrolytlösung ist.

28. Gebrauch nach einem der vorhergehenden Ansprüche, wobei das Medikament in zwei Dosierzyklen zu verabreichen ist.

29. Gebrauch nach Anspruch 28, wobei jeder Dosierzyklus (i) eine siebentägige Verabreichungsphase, in der vier 250-ml-Teildosen einer 2 %igen Taurolidinlösung über jeden der sieben Tage als Dauerinfusion zu verabreichen sind, und (ii) eine siebentägige Nichtverabreichungsphase umfasst.

30. Gebrauch nach einem der Ansprüche 1 bis 27, wobei das Medikament in vier Dosierzyklen zu verabreichen ist.

31. Gebrauch nach Anspruch 30, wobei jeder Dosierzyklus eine siebentägige Verabreichungsphase umfasst, bei der an jedem Tag vier 250-ml-Teildosen einer 2 %igen Taurolidinlösung derart zu verabreichen sind, dass jede Teildosis über einen Zeitraum von zwei Stunden, gefolgt von einer Nichtverabreichungspause von einer Stunde zu infundieren ist.

32. Taurolidin, Taurultam oder ein Gemisch aus diesen zum Gebrauch beim Einleiten von apoptotischem Tod einer neoplastischen Zelle in einem Säuger durch Inberührungbringen der Zelle mit einer Apoptose einleitenden Menge der Taurolidinverbindung, der Taurultamverbindung oder eines Gemischs aus diesen, wobei die Zusammensetzung in wenigstens zwei Dosierzyklen zu verabreichen ist, wobei jeder Dosierzyklus (i) eine 1 bis 8 Tage lange Verabreichungsphase, in der die Verbindung an jedem Tag mit einer Tagesgesamtdosis von 2 bis 60 g zu verabreichen ist, und (ii) eine 1 bis 14 Tage lange Nichtverabreichungsphase, in der keine Taurolidinverbindung, keine Taurultamverbindung und kein Gemisch aus diesen verabreicht wird, umfasst.

33. Taurolidin, Taurultam oder ein Gemisch aus diesen nach Anspruch 32 zum Gebrauch beim Einleiten von apoptotischem Tod einer neoplastischen Zelle in einem Säuger nach Anspruch 32, wobei das Inberührungbringen mit der neoplastischen Zelle nach einem der Ansprüche 2 bis 31 ausgeführt wird.

## Revendications

1. Utilisation d'un agent de transfert de méthylol dans la fabrication d'un médicament destiné à être utilisé pour induire la mort par apoptose d'une cellule néoplasique chez un mammifère par le contact de ladite cellule avec une quantité induisant l'apoptose dudit médicament, dans laquelle ledit médicament doit être administré pendant au moins deux cycles de dosage, chaque cycle de dosage comprenant (i) une phase d'administration de 1 à 8 jours pendant laquelle ledit médicament est administré chaque jour à une dose quotidienne totale de 2 g à 60 g dudit agent de transfert de méthylol, et (ii) une phase de non-administration de 1 à 14 jours pendant laquelle aucun agent de transfert de méthylol n'est administré, dans laquelle l'agent de transfert de méthylol est la taurolidine, le taurultam ou un mélange de ceux-ci.

2. Utilisation selon la revendication 1, dans laquelle l'agent de transfert de méthylol est la taurolidine.

3. Utilisation selon la revendication 2, dans laquelle le médicament doit être administré pendant chaque phase d'administration à une dose quotidienne totale de 2 g à 30 g de taurolidine.

4. Utilisation selon la revendication 1, dans laquelle l'agent de transfert de méthylol est le taurultam.

5. Utilisation selon la revendication 4, dans laquelle le médicament doit être administré pendant chaque phase d'administration à une dose quotidienne totale de 4 g à 60 g de taurultam.

6. Utilisation selon l'une quelconque des revendications précédentes, dans laquelle le médicament doit être administré pendant chaque phase d'administration à une dose qui fournit 150 mg/kg à 450 mg/kg par jour dudit agent de transfert de méthylol.

7. Utilisation selon la revendication 6, dans laquelle le médicament doit être administré pendant chaque phase d'administration à une dose qui fournit 300 mg/kg à 450 mg/kg par jour dudit agent de transfert de méthylol.

8. Utilisation selon l'une quelconque des revendications précédentes, dans laquelle le médicament doit être coadministré avec un second agent antinéoplasique.

9. Utilisation selon la revendication 8, dans laquelle le second agent antinéoplasique est le 5-fluorouracile.

10. Utilisation selon la revendication 9, dans laquelle le 5-fluorouracile doit être administré en une quantité quotidienne de 100 mg/m² à 5000 mg/m² de surface corporelle.

11. Utilisation selon la revendication 10, dans laquelle le 5-fluorouracile doit être administré en une quantité quotidienne de 200 mg/m² à 1000 mg/m² de surface corporelle.

12. Utilisation selon l'une quelconque des revendications 1 à 7, dans laquelle le médicament doit être coadministré avec le ligand de Fas.

13. Utilisation selon la revendication 12, dans laquelle le ligand de Fas doit être administré en une quantité quotidienne de 0,01 mg/kg à 1000 mg/kg de poids corporelle.

14. Utilisation selon l'une quelconque des revendications précédentes, dans laquelle le médicament comprend une solution de l'agent de transfert de méthylol, ladite solution contenant en outre de la taurine.

15. Utilisation selon la revendication 14, dans laquelle ladite taurine est présente dans ladite solution à une concentration située dans la plage allant de 1 g/l à 10 g/l.

16. Utilisation selon l'une quelconque des revendications précédentes, dans laquelle le médicament est destiné à être utilisé dans le traitement ou la prophylaxie d'un cancer hépatique.

17. Utilisation selon la revendication 16, dans laquelle le médicament doit être administré directement au foie par l'intermédiaire d'un vaisseau hépatique.

18. Utilisation selon la revendication 16, dans laquelle le médicament doit être administré par l'intermédiaire d'une artère hépatique ou d'une artère gastroduodénale lors du traitement d'une tumeur hépatique primaire.

19. Utilisation selon la revendication 17, dans laquelle le médicament doit être administré par l'intermédiaire d'une veine porte lors du traitement d'un cancer hépatique métastatique.

20. Utilisation selon l'une quelconque des revendications précédentes, dans laquelle le médicament doit être administré pendant chaque phase d'administration par perfusion continue de façon que la dose quotidienne de l'agent de transfert de méthylol soit perfusée sur une période de 24 heures.

21. Utilisation selon l'une quelconque des revendications 1 à 19, dans laquelle le médicament doit être administré pendant chaque phase d'administration par perfusion de façon que la dose quotidienne de l'agent de transfert de méthylol soit perfusée sous la forme d'une série de doses partielles, chaque perfusion de dose partielle étant suivie d'une pause pendant laquelle aucune perfusion n'est effectuée.

22. Utilisation selon la revendication 21, dans laquelle les doses partielles doivent être perfusées de façon que la dose quotidienne totale de l'agent de transfert de méthylol soit administrée sur une période de 24 heures.

23. Utilisation selon la revendication 22, dans laquelle chaque dose partielle doit être perfusée sur une période de deux heures, qui est suivie d'une pause de quatre heures.

24. Utilisation selon la revendication 21, dans laquelle les doses partielles doivent être perfusées de façon que la dose quotidienne totale de l'agent de transfert de méthylol soit administrée sur une période de moins de 24 heures.

25. Utilisation selon la revendication 24, dans laquelle chaque dose partielle doit être perfusée sur une période d'une heure, qui est suivie d'une pause d'une heure.

26. Utilisation selon l'une quelconque des revendications précédentes, dans laquelle ledit médicament doit être coadministré avec au moins un agent supplémentaire choisi parmi les anticonvulsivants, les agents anti-oedème, les agents antibactériens et les solutions d'électrolyte.

27. Utilisation selon la revendication 26, dans laquelle l'agent supplémentaire est une solution d'électrolyte.

28. Utilisation selon l'une quelconque des revendications précédentes, dans laquelle le médicament doit être administré lors de deux cycles de dosage.

29. Utilisation selon la revendication 28, dans laquelle chaque cycle de dosage comprend (i) une phase d'administration de sept jours pendant laquelle quatre portions de dose de 250 ml d'une solution de taurolidine à 2 % doivent être administrées sous la forme d'une perfusion continue pendant chaque jour des sept jours, et (ii) une phase de non-administration de sept jours.

30. Utilisation selon l'une quelconque des revendications 1 à 27, dans laquelle le médicament doit être administré lors de quatre cycles de dosage.

31. Utilisation selon la revendication 30, dans laquelle chaque cycle de dosage comprend une phase d'administration de sept jours pendant laquelle, chaque jour, quatre portions de dose de 250 ml d'une solution de taurolidine à 2 % doivent être administrées de façon que chaque portion de dose soit perfusée sur une période d'une à deux heures, qui est suivie d'une pause sans administration d'une heure.

32. Taurolidine, taurultam ou mélange de ceux-ci, pour une utilisation dans l'induction de la mort par apoptose d'une cellule néoplasique chez un mammifère par le contact de ladite cellule avec une quantité induisant l'apoptose dudit composé de taurolidine, dudit composé de taurultam ou d'un mélange de ceux-ci, dans lequel ledit composé doit être administré pendant au moins deux cycles de dosage, chaque cycle de dosage comprenant (i) une phase d'administration de 1 à 8 jours pendant laquelle ledit composé est administré chaque jour à une dose quotidienne totale de 2 g à 60 g, et (ii) une phase de non-administration de 1 à 14 jours pendant laquelle aucun composé de taurolidine, aucun composé de taurultam ou aucun mélange de ceux-ci n'est administré.

33. Taurolidine, taurultam ou mélange de ceux-ci selon la revendication 32, pour une utilisation dans l'induction de la mort par apoptose d'une cellule néoplasique chez un mammifère selon la revendication 32, dans lequel ledit contact avec ladite cellule néoplasique est tel que défini dans l'une quelconque des revendications 2 à 31.
